# EUROPEAN PATENT SPECIFICATION

(11) **EP 1 993 450 B1**
(45) Date of publication and mention of the grant of the patent: **18.08.2010**
(21) Application number: 07713355.1
(22) Date of filing: 14.03.2007
(51) Int. Cl.: A61B 17/04, A61B 17/28

(54) **SURGICAL INSTRUMENT FOR ATTACHING SOFT TISSUE TO A BONE**
CHIRURGISCHES INSTRUMENT ZUR BEFESTIGUNG VON WEICHEM GEWEBE AN KNOCHEN
INSTRUMENT CHIRURGICAL DE FIXATION D'UN TISSU MOU A UN OS

(30) Priority: 17.03.2006 US 783035 P
(43) Date of publication of application: 26.11.2008
(73) Proprietor: T.A.G. Medical Products a Limited Partnership, 25130 Doar Na Oshrat (IL); Oren, Ran, 25 130 Doar Na Oshrat (IL); Moor, Dan, 25 130 Doar Na Oshrat (IL)
(72) Inventor: OREN, Ran, 25130 Doar-na Oshrat (IL); MOOR, Dan, 25130 Doar-na Oshrat (IL); RANON, Lee, 22407 Nahariya (IL); ZAKAI, Eran, 20180 Doar-na Misgav (IL); LAFOSSE, Laurent, F-74940 Annecy-le-vieux (FR)
(74) Representative: Machtalère, Georges
(86) International application number: PCT/IL2007/000335
(87) International publication number: WO 2007/107980

(56) References cited:
- US-A- 4 836 205
- US-A- 5 683 401
- US-A1- 2004 138 682
- US-A1- 2005 043 748

## Description

### FIELD AND BACKGROUND OF THE INVENTION

The present invention relates to a surgical instrument for attaching soft tissue to a bone. The invention is particularly useful, and is therefore described below, with respect to the repair of labral tears in shoulder joints, in which the labrum of the shoulder joint is to be surgically reattached to the glenoid bone, but it will be appreciated that the invention could advantageously be used in other applications as well.

Minimally-invasive tissue repair procedures, such as arthroscopic and endoscopic procedures, are generally preferred whenever possible in order to minimize trauma and damage to surrounding tissue layers, thus shortening the time needed for recovery. This is particularly true with respect to the repair of labral tears in shoulder joints.

The range of movements the human shoulder can make far exceeds any other joint in the body. The shoulder joint is a ball and socket joint, similar to the hip; however, the socket of the shoulder joint is extremely shallow, and thus inherently unstable. Muscles and tendons serve to keep the bones in approximation. In order to compensate for the shallow socket, the shoulder joint has a cuff of fibrous cartilage called a labrum that forms a cup for the head of the arm bone (humerus) to move within. This cuff of cartilage makes the shoulder joint much more stable, yet allows for a very wide range of movements. When the labrum of the shoulder joint is damaged, the stability of the shoulder joint is compromised.

Shoulder dislocations often tear the labrum, especially in younger patients. The labrum tear, called a Bankart lesion, in most cases involves the part of the labrum called the inferior glenohumeral ligament. The lesion is seen in over 85% of cases after a traumatic anterior dislocation. The inferior gleno-humeral ligament, which is attached medially to the lower half of the anterior glenoid labrum, is the most important of the ligaments that stabilize the shoulder. At the time of the original injury, the humeral head, when it is forced out anteriorly and inferiorly, first stretches the anterior capsule and the inferior glenohumeral ligament. Then, as a result of traction, the fibrous labrum is pulled off from the inferior half of the anterior rim of the glenoid. The damage suffered, if not treated, may cause recurring dislocations,

With past mid-age sedentary patients, conventional treatment may be recommended; but with younger and physically active patients, surgical intervention is usually necessary to restore the stability of the shoulder joint to full function. The aim of the operation is to re-attach the separated part of the labrum to the glenoid at its normal anatomical position.

The procedure for repairing a labral tear arthroscopically involves the following steps:
(1) mapping the joint and opening portals for visualization, irrigation and for the instruments through small cuts in the skin;
(2) drilling bores in the glenoid rim;
(3) inserting bone anchors with sutures attached to each anchor into the bores;
(4) grasping the torn labrum and moving it back into its original position on the glenoid; and
(5) bringing suture strands coming from the anchor through the tissue to the outside using a suture retriever and tying the strands to attach and tighten the labrum to the glenoid rim.

Several prior art devices are available to perform each step of the process separately: anchor inserters, graspers, suture retrievers, etc. Examples of such prior art devices are described in US Patents 4,836,205, 5,683,401, 6,511,487 and 5,499,991 and in US Published Patent Applications US2004/138682A1, US2005/0043748A1 and US 2002/0065526.

### OBJECTS AND BRIEF SUMMARY OF THE PRESENT INVENTION

An object of the present invention is to provide a single surgical instrument capable of performing all the necessary steps in attaching soft tissue to a bone in a surgical site, particularly steps (3)-(5) in the above-described procedure for repairing a labral tear. A method of attaching soft tissue to a bone is provided which method is particularly useful in the above-described procedure for repairing labral tears in shoulder joints.

According to one aspect of the present invention, there is provided a surgical instrument for use in attaching soft tissue to a bone in a surgical site, comprising: an elongated shaft having a distal end carrying a clamping device including clamping jaws, and a piercing device for piercing soft tissue; the elongated shaft having a proximal end carrying manipulatable members for manipulating the clamping device to clamp soft tissue between the clamping jaws, and for manipulating the piercing device for piercing the soft tissue when clamped between the clamping jaws, and for drawing a suture through the pierced soft tissue; **characterized in that** said elongated shaft is rigid from its proximal end to its distal end, and further **characterized in that** the surgical instrument further includes, at the distal end of the elongated shaft, a socket dimensioned to receive an anchor to be implanted in a bore in the bone, which anchor also has secured thereto a suture to be passed through the pierced soft tissue and to be tied to the bone.

The surgical instrument and method are described below with respect to a preferred embodiment for the repair of labral tears wherein the tissue to be attached is a portion of the labrum separated detached from the glenoid bone in a shoulder joint.

Further features and advantages of the invention will be apparent from the description below.

### BRIEF DESCRIPTION OF THE DRAWINGS

The invention is herein described, by way of example only, with reference to the accompanying drawings, wherein:
Fig. 1 schematically illustrates the anatomy of a shoulder joint;
Fig. 2 is a side elevational view illustrating one form of surgical instrument constructed in accordance with the present invention;
Fig. 3 is an enlarged fragmentary view of the distal end of the surgical instrument of Fig. 2;
Fig. 4 illustrates the distal end of the surgical instrument of Fig. 3 in a particular stage of its use, wherein labral tissue, clamped between the jaws of the instrument, is about to be pieced by the piercing device of the surgical instrument;
Fig. 5 schematically illustrates one technique of suturing using the surgical instrument of the Figs. 2-4; and
Fig. 6 illustrates an alternative method of suturing using the suturing instrument of Figs. 2-4.

It is to be understood that the foregoing drawings, and the description below, are provided primarily for purposes of facilitating understanding the conceptual aspects of the invention and possible embodiments thereof, including what is presently considered to be a preferred embodiment. In the interest of clarity and brevity, no attempt is made to provide more details than necessary to enable one skilled in the art, using routine skill and design, to understand and practice the described invention. It is to be further understood that the embodiments described are for purposes of example only, and that the invention is capable of being embodied in other forms and applications than described herein.

### THE ANATOMY OF A SHOULDER JOINT

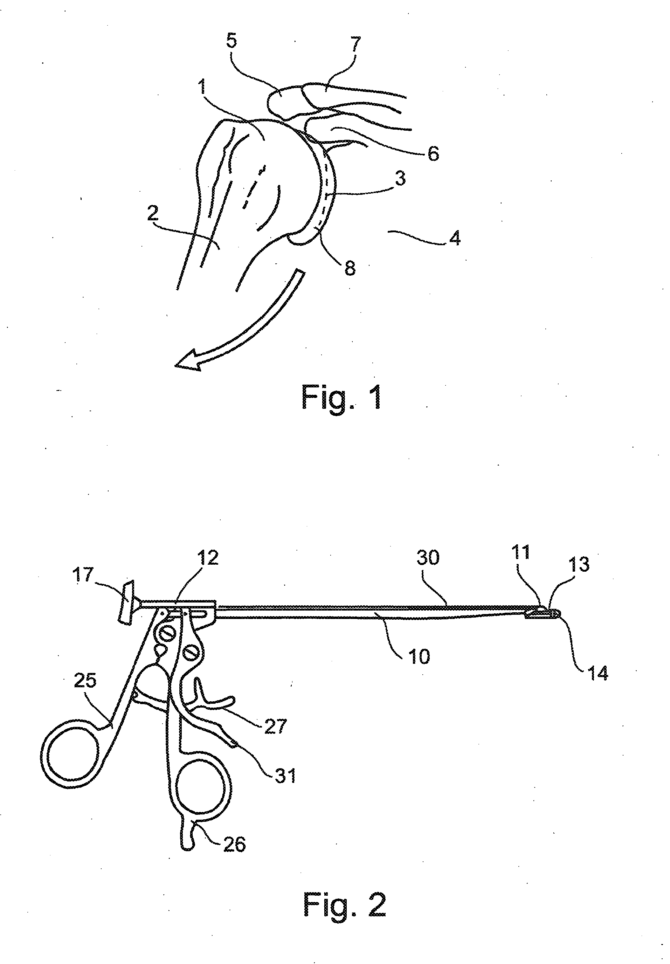

Fig. 1 illustrates the anatomy of a shoulder joint. The head 1 of the upper arm bone or humerus 2, forms a ball-and-socket joint with the shallow glenoid cavity 3. The glenoid is the lateral part of the shoulder blade scapula 4. Two hook-like projections of the scapula overhanging the glenoid are the acromion 5 and the coracoid process 6. A group of muscles, collectively know as the Rotator Cuff, originate on the scapula and insert on the humerus. These serve to stabilize the joint by keeping the humeral head in contact with the glenoid cavity. The clavicle 7 connects the acromion to the breastbone sternum. The glenoid labrum 8, which is a flexible fibrous ligament, surrounds the glenoid rim enlarging its area of contact with the humerus. When dislocations in the direction shown by the arrow occur, the anterior-inferior part of the labrum is torn away from the glenoid, causing instability of the joint.

Portals to be used in the repair procedure are placed relative to the bony structures marked out on the skin. The portals must provide safe access to the surgical site at an angle suitable for work.

Of the various steps involved in the procedure for repairing a labral tear arthroscopically as briefly described above, the surgical instrument illustrated in Figs. 2-4 of the drawings is particularly useful for performing steps (3)-(5).

### DESCRIPTION OF A PREFERRED EMBODIMENT

### The Overall Construction

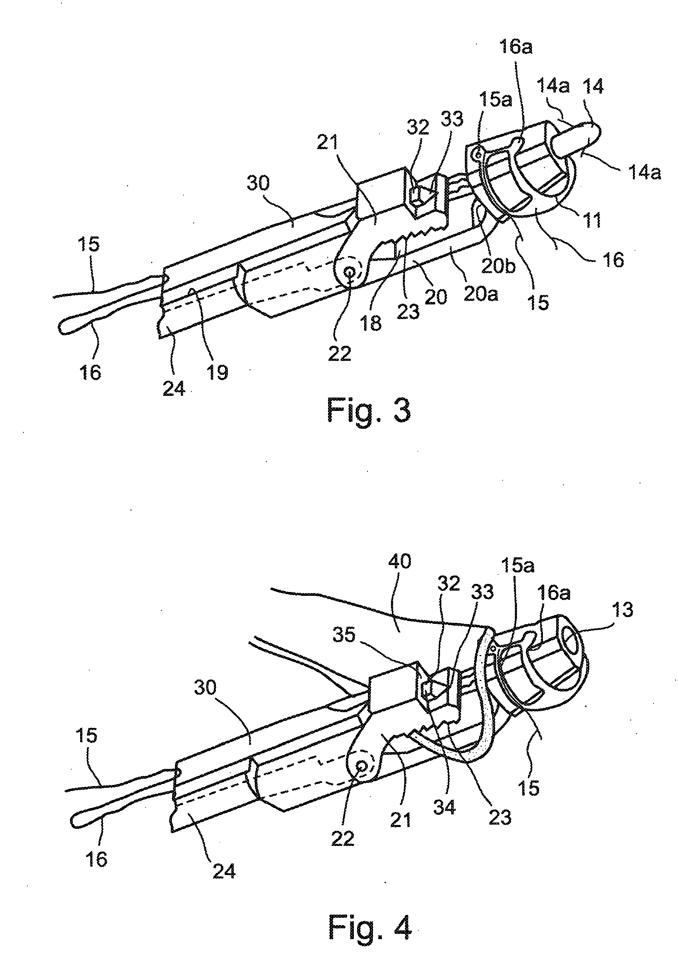

Fig. 2 illustrates one form of surgical instrument constructed in accordance with the present invention; whereas Figs. 3 and 4 illustrate the distal end of the instrument.

As shown in Fig. 2, the surgical instrument includes an elongated shaft 10 having a distal end 11 for insertion into the surgical site, and a proximal end 12 to be located outside of the surgical site for manipulating the surgical instrument and the various parts thereof, as will be described more particularly below.

Elongated shaft 10 is made of a rigid material, e.g., metal, of a suitable cross-section. Its distal tip is formed with a socket 13 (Fig. 4) dimensioned to receive an anchor 14 (Fig. 3), to be force-fitted into a bore formed in the bone (glenoid) to which soft tissue (fibril tissue) is to be attached. As shown in Fig. 3, anchor 14 includes barbs 14a, 14b, for securely anchoring it in place. In addition, the anchor includes two sutures 15, 16, to be used for attaching the labral tissue to the bone receiving the anchor.

The distal tip 11 of elongated shaft 10 is further formed with two slots 15a, 16a, for receiving the free ends of the two sutures, 15, 16, respectively, fixed to the anchor 14.

The proximal end 12 of elongated shaft 10 is further provided with a finger-piece 17 to be used, as will be described below, for force-fitting the anchor 14 into a bore formed in the bone to which the soft tissue is to be attached. It will be seen in Fig. 2 that the longitudinal axis of the finger-piece 17 and of the proximal portion 12 of elongated shaft 10 is substantially parallel to the longitudinal axis of the socket 13 at the distal end 11 of the elongated shaft. This facilitates the force-fitting of the anchor 14 into the bore in the bone by an axial pressure applied, e.g., via the user's thumb, to the finger-piece 17.

Elongated shaft 10 is further formed, at its distal end, with a recess 18 immediately proximally to the two slots 15a, 16a receiving the sutures 15, 16. As will be described below, recess 18 in elongated shaft 10 defines one jaw of a clamping device carried at the distal end of the elongated shaft for engaging and clamping a portion of the soft tissue (e.g., labral tissue) to be attached to the bone in the surgical site.

Elongated shaft 10 is further formed with a channel 19 on its upper face extending from its proximal end 12 to its distal end 11. As will be described below, channel 19 accommodates a piercing device, in the form of a crochet head, used for piercing the portion of the soft tissue clamped by the clamping device, and for drawing therethrough one of the sutures 15, 16 from the anchor 14.

As shown particularly in Fig. 3, recess 18 at the distal end 11 of elongated shaft 10 is shaped to define a fixed jaw 20 having an axially-extending portion 20a and a transversely-extending portion 20b at the distal end of the recess. The distal end of elongated shaft 10 further includes a pivotal jaw 21 pivotally mounted at 22, at the proximal end of recess 18, to a closed or clamping position with respect to fixed jaw 20, or to an open or unclamping position with respect to the fixed jaw. The under-face of pivotal jaw 21 is grooved or serrated, as shown at 23, to firmly clamp the soft tissue therebetween when the clamping device is in its closed, clamping condition.

Pivotal jaw 21 is coupled to one end of a link 24 extending the along the length of the elongated shaft 10. The opposite end of link 24 is coupled to a handle 25 pivotally mounted at the proximal end of the elongated shaft. Handle 25 cooperates with a second handle 26 fixed to the elongated shaft at its proximal end, such that moving handle 25 towards handle 26 pivots jaw 21 towards the fixed jaw 20 to clamp any soft tissue therebetween, and moving handle 25 away from handle 26 pivots jaw 21 to its open position to release the tissue.

As further shown in Fig. 2, the proximal end of elongated shaft 10 further includes a ratchet arm 27 pivotally carried by handle 25 and engageable with handle 26, to releasably retain the two handles in any moved position, and thereby to releasably retain any soft tissue clamped between the two jaws 20, 21 irrespective of the thickness of the tissue.

The piercing device 30 included in the illustrated surgical instrument is in the form of an elongated member (Figs. 3, 4) which is longitudinally movable within channel 19 formed in the upper face of elongated shaft 10 in the distal and proximal directions. For this purpose, elongated member of piercing device 30 is coupled, at the proximal end of the elongated shaft, to a pivotal arm 31 (Fig. 2), such that moving arm 31 towards handle 25, pushes the piercing device 30 in the distal direction, and moving arm 31 away from handle 25 pulls the piercing device 30 in the proximal direction.

Piercing device 30 is used for piercing the tissue clamped between jaws 20 and 21 of the clamping device at the distal end of the surgical instrument, and for drawing a suture through the hole so formed. Thus, as shown in Figs. 3 and 4, the distal end of piercing device 30 carries a crochet head 32 formed with a pointed tip 33 for piercing the tissue clamped between the jaws 20, 21 during the movement of the piercing device in the distal direction. Crochet head 32 is further formed with a hook formation 34 for engaging one of the sutures 15, 16, and for drawing same back through the so-formed pierced opening during the movement of the piercing device in the proximal direction. The movements of the piercing device 30 are guided by a channel 35 formed in the upper surface of pivotal jaw 21.

### Examples of the Manner of Use

As indicated earlier, the surgical instrument illustrated in Figs. 2-4 may be used in performing steps 3-5 described above for repairing a labral tear arthroscopically. Steps 1 and 2 are performed in a conventional manner. The appropriate number of bores would be drilled in the glenoidal rim, each bore receiving one of the anchors 14 (Fig. 3) to be used in the surgical procedure.

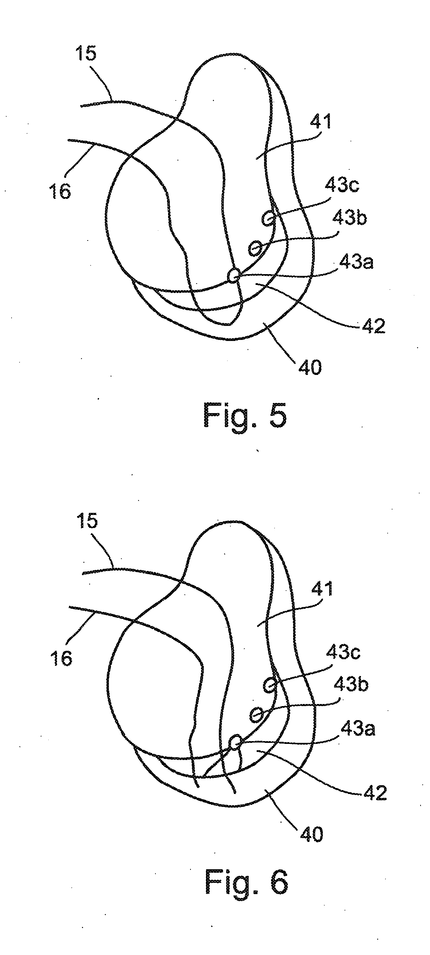

For each such bore, the surgical instrument is loaded with the appropriate anchor 14 inserted into socket 13 at the distal end of elongated shaft 10, and its sutures 15, 16 are received within their respective slots 15a, 16a at the distal end of the elongated shaft. In the example described herein, the soft tissue to be attached is pierced at two points for each anchor to receive the two sutures 15, 16, as shown in Fig. 6. It will be appreciated, however, that in some applications it may be sufficient to pierce the tissue at only one point, in which case only one suture, e.g., 15, would be drawn through the pierced opening in the tissue to be attached.

Fig. 5 schematically illustrates the labral tissue 40 separated from the glenoid 41, because of a labral tear, producing a gap 42, which is to be arthroscopically repaired by inserting anchors 14 into bores formed in the glenoid 41, passing sutures through the fibril tissue and tying same to the glenoid. Fig. 5 illustrates three bores 43a-43c for receiving three anchors 14, but it will be appreciated that any appropriate number of anchors can be used according to the particular condition to be repaired.

After the illustrated surgical instrument is loaded with the first anchor 14, the anchor is inserted into the lower-drilled bore 43a, as shown in Fig. 5, and is fixed therein by applying pressure to finger-piece 17 at the proximal end of the instrument.

Jaws 20, 21 of the clamping device are now opened via handles 25, 26, and the instrument is positioned within the shoulder joint so that the fixed jaw 20 lies under the inferior separated part of the labrum 40, while the movable jaw 21 is above the surface of the labrum.

The jaws are then closed, whereupon the labral tissue 40 is grasped between the jaws and is folded against the distal shoulder 20b defined by the recess 18 formed in the fixed jaw member 20 (Fig. 4). The tissue is then manipulated to lie over its original anatomic position.

With the device held in this position, the surgeon advances piercing device 30 in the distal direction by actuating lever 31 to cause the pointed tip 33 of the crochet head 32 to pierce the tissue. The piercing device then executes the return movement, in the proximal direction, whereupon hook formation 34 of the crochet head 32 engages suture strand 15 in slot 15a and pulls it through the tissue. Opening the jaws 20, 21 now allows the surgeon to disengage the device from the labrum and bring it outside of the body for knotting suture strands 15, 16 with a sliding knot, which is then manipulated to tighten the labrum to the glenoid.

As shown in Fig. 5, suture strand 15 comes directly from the anchor 14, and passes through the tissue 40. Thus, when the sliding knot tightens the labrum to the glenoid, the gap 42 formed between the glenoid 41 and the separated labrum 40 is closed.

In the alternative method of suturing shown in Fig. 6, both suture strands 15, 16 are loaded into their respective slots 15a and 16a respectively. After the first suture strand 15 is brought through the tissue 40 as described above, the jaws are opened. The surgeon now grasps the labrum again at a suitable distance from the exit point of the first suture strand 15 and operates the piercing device 30 to pierce the tissue with a second hole, and to bring the second strand 16 from the second slot 16a through that hole in the tissue. The knot tied between these sutures overlies the labrum as both strands now come through the labrum.

The above procedure is repeated for each anchor to be inserted. It will be seen - that the surgical instrument need be removed from the surgical site only when inserting another anchor.

While the invention has been described to one preferred embodiment, it will be appreciated that this is set forth merely for purposes of example, and that many other variations, modifications and applications of the invention may be made.

## Claims

1. A surgical instrument for use in attaching soft tissue to a bone in a surgical site, comprising:
an elongated shaft (10) having a distal end (11) carrying a clamping device including clamping jaws (20, 21), and a piercing device (30) for piercing soft tissue;
said elongated shaft (10) having a proximal (12) end carrying manipulatable members (26, 27) for manipulating said clamping device to clamp soft tissue between the clamping jaws (20, 21), and for manipulating said piercing device (30) for piercing the soft tissue (40) when clamped between said clamping jaws (20,21), and for drawing a suture (15,16) through the pierced soft tissue (40);
**characterized in that** said elongated shaft (10) is rigid from its proximal end (12) to its distal end(11), and
that said surgical instrument further includes, at the distal end (11) of said elongated shaft (10), a socket (13) dimensioned to receive an anchor (14) to be implanted in a bore (43a, 43b, 43c) in the bone (43), which anchor (14) also has secured thereto a suture (15, 16) to be passed through the pierced soft tissue (40) and to be tied to the bone (43).

2. The surgical instrument according to Claim 1, wherein said piercing device (30) includes a crochet head (32) having a pointed distal tip (33) for piercing tissue (40) clamped between the jaws (20, 21) of said clamping device during the movement of the crochet head (32) in the distal direction.

3. The surgical instrument according to Claim 2, wherein said crochet head (32) has a hook formation (34) spaced from its pointed distal tip (33) for receiving the suture (15, 16), and for drawing same through the pierced tissue (40) during the movement of the crochet head (32) in the proximal direction.

4. The surgical instrument according to Claim 1, wherein said distal end (12) of the elongated shaft (10) is formed with a slot (15e) extending across the path of movement of the piercing device (30) when moving in the proximal direction, for receiving the suture (15) to be drawn through a hole formed in the tissue (40) after pierced by said piercing device (30).

5. The surgical instrument according to Claim 4, wherein said distal end (12) of the elongated shaft (10) is formed with a second slot (16a) for receiving a second suture (16) secured to the anchor(14), to be drawn through a second hole formed by said piercing device.

6. The surgical instrument according to Claim 1, wherein said clamping jaws (20,21) include a fixed jaw (20) fixed to said elongated shaft (10), and a pivotal jaw (21) pivotal towards and away from said fixed jaw (20) to closed and open positions with respect thereto.

7. The surgical instrument according to Claim 6, wherein said piercing device (30) moves through a channel (19) formed in said fixed jaw (20).

8. The surgical instrument according to Claim 1, wherein the longitudinal axis of said socket (14) at said distal end (11) of the elongated shaft (10) is parallel to the longitudinal axis of said elongated shaft (10); and wherein the proximal end (12) of the elongated shaft (10) includes a finger-piece (17) having an axis also parallel to the longitudinal axis of said elongated shaft for pressure-forcing an anchor (14) in said socket (13) into a bore (43 a) formed in the bone (41).

9. The surgical instrument according to Claim 1, wherein said manipulatable members carried at the proximal end (12) of said elongated shaft (10) include: a first handle (26) fixed to the proximal end (12) of said elongated shaft (10), and a second handle (25) pivotal to the proximal end (12) of said elongated shaft and coupled to one (21) of said clamping jaws (20, 21) for moving same to closed and open position with respect to said fixed jaw (20) upon pivoting said second handle (25) with respect to said first handle (26).

10. The surgical instrument according to Claim 9, wherein said manipulatable members carried at the proximal end of said elongated shaft (10) further includes a lever arm (31) pivotal to said first handle (26) and coupled to said piercing device (30) for moving same in the distal and proximal directions upon pivoting said lever arm (31) with respect to said first handle (26).

11. The surgical instrument according to Claim 9, wherein said manipulatable members carried by said proximal end of the elongated shaft (10) further includes a ratchet device (27) for releasable retaining said first (26) and second (25) handles in a selected pivoted position with respect to each other.

12. The surgical instrument according to Claim 1, wherein said elongated shaft (10), clamping device (20-23) and piercing device (30) are dimensioned for repairing a labrum tear in a shoulder joint.

## Patentansprüche

1. Chirurgisches Instrument zur Verwendung bei der Befestigung von weichem Gewebe an einem Knochen an einer Operationsstelle, umfassend:
einen länglichen Schaft (10) mit einem distalen Ende (11), das eine Klemmvorrichtung, die Klemmbacken (20,21) beinhaltet, trägt, und einer Durchstechvorrichtung (30) zum Durchstechen von weichem Gewebe;
wobei der längliche Schaft (10) ein proximales Ende (12) hat, das betätigbare Elemente (26, 27) trägt, zum Betätigen der Klemmvorrichtung, um weiches Gewebe zwischen den Klemmbacken (20, 21) einzuklemmen, und zum Betätigen der Durchstechvorrichtung (30) zum Durchstechen des weichen Gewebes (40), wenn es zwischen den Klemmbacken (20, 21) eingeklemmt ist, und zum Hindurchziehen eines chirurgischen Fadens (15, 16) durch das durchstochene weiche Gewebe (40);
**dadurch gekennzeichnet, dass** der längliche Schaft (10) von seinem proximalen Ende (12) bis zu seinem distalen Ende (11) starr ist, und
dass das chirurgische Instrument weiter an dem distalen Ende (11) des länglichen Schafts (10) eine Halterung (13) beinhaltet, die zur Aufnahme eines in einer Bohrung (43a, 43b, 43c) in dem Knochen (43) zu implantierenden Ankers (14) dimensioniert ist, an welchem Anker (14) auch ein chirurgischer Faden (15, 16) gesichert ist, der durch das durchstochene weiche Gewebe (40) geführt und an dem Knochen (43) angebunden werden soll.

2. Chirurgisches Instrument nach Anspruch 1, wobei die Durchstechvorrichtung (30) einen Häkelkopf (32) umfasst, der eine zugespitzte distale Spitze (33) zum Durchstechen von zwischen den Backen (20, 21) der Klemmvorrichtung eingeklemmtem Gewebe (40) während der Bewegung des Häkelkopfes (32) in der distalen Richtung aufweist.

3. Chirurgisches Instrument nach Anspruch 2, wobei der Häkelkopf (32) eine von seiner zugespitzten distalen Spitze (33) beabstandete Hakenformation (34) zur Aufnahme des chirurgischen Fadens (15, 16) und zum Hindurchziehen desselben durch das durchstochene Gewebe (40) während der Bewegung des Häkelkopfs (32) in der proximalen Richtung aufweist.

4. Chirurgisches Instrument nach Anspruch 1, wobei das distale Ende (12) des länglichen Schafts (10) mit einem Schlitz (15e) ausgebildet ist, der sich quer zur Bewegungsbahn der Durchstechvorrichtung (30) beim Bewegen in der proximalen Richtung erstreckt, zur Aufnahme des chirurgischen Fadens (15), der durch ein in dem Gewebe (40) gebildetes Loch gezogen werden soll, nachdem dieses Gewebe von der Durchstechvorrichtung (30) durchstochen wurde.

5. Chirurgisches Instrument nach Anspruch 4, wobei das distale Ende (12) des länglichen Schafts (10) mit einem zweiten Schlitz (16a) zur Aufnahme eines zweiten, an dem Anker (14) befestigten chirurgischen Fadens (16) ausgebildet ist, der durch ein zweites, von der Durchstechvorrichtung gebildetes Loch gezogen werden soll.

6. Chirurgisches Instrument nach Anspruch 1, wobei die Klemmbacken (20, 21) eine an dem länglichen Schaft (10) fixierte feststehende Backe (20) und eine schwenkbare Backe (21), die zu der feststehenden Backe (20) hin und davon weg in eine geschlossene und offene Position in Bezug dazu schwenkbar ist, beinhalten.

7. Chirurgisches Instrument nach Anspruch 6, wobei die Durchstechvorrichtung (30) sich durch einen in der feststehenden Backe (20) ausgebildeten Kanal (19) bewegt.

8. Chirurgisches Instrument nach Anspruch 1, wobei die Längsachse der Halterung (14) an dem distalen Ende (11) des länglichen Schafts (10) parallel zur Längsachse des länglichen Schafts (10) ist; und wobei das proximale Ende (12) des länglichen Schafts (10) ein Fingerteil (17) umfasst, das eine Achse aufweist, die ebenfalls parallel zur Längsachse des länglichen Schafts ist, um einen Anker (14) in der Halterung (13) mit Druck in eine in dem Knochen (41) gebildete Bohrung (43a) zu zwingen.

9. Chirurgisches Instrument nach Anspruch 1, wobei die an dem proximalen Ende (12) des länglichen Schafts (10) getragenen betätigbaren Elemente beinhaltet einen an dem proximalen Ende (12) des länglichen Schafts (10) fixierten ersten Handgriff (26), und einen zweiten Handgriff (25), der schwenkbar an dem proximalen Ende (12) des länglichen Schafts ist und an eine (21) der Klemmbacken (20, 21) gekoppelt ist, um selbige, bei Schwenken des zweiten Handgriffs (25) in Bezug auf den ersten Handgriff (26), in eine geschlossene und offene Position bezüglich der feststehenden Backe (20) zu bewegen.

10. Chirurgisches Instrument nach Anspruch 9, wobei die an dem proximalen Ende (12) des länglichen Schafts (10) getragenen betätigbaren Elemente weiter einen Hebelarm (31) beinhalten, der schwenkbar an dem ersten Handgriff (26) ist und an die Durchstechvorrichtung (30) gekoppelt ist, um selbige, bei Schwenken des Hebelarms (31) in Bezug auf den ersten Handgriff (26), in die distale und proximale Richtung zu bewegen.

11. Chirurgisches Instrument nach Anspruch 9, wobei die von dem proximalen Ende des länglichen Schafts (10) getragenen betätigbaren Elemente weiter eine Sperrklinkenvorrichtung (27) zum lösbaren Festhalten des ersten (26) und des zweiten (25) Handgriffs in einer ausgewählten geschwenkten Position in Bezug zueinander beinhalten.

12. Chirurgisches Instrument nach Anspruch 1, wobei der längliche Schaft (10), die Klemmvorrichtung (20-23) und die Durchstechvorrichtung (30) zum Reparieren eines Labrumrisses in einem Schultergelenk dimensioniert sind.

## Revendications

1. Instrument chirurgical à utiliser pour fixer un tissu mou à un os dans un site chirurgical, comprenant :
une tige allongée (10) possédant une extrémité distale (11) supportant un dispositif de serrage englobant des mâchoires de serrage (20, 21) et un dispositif de perçage (30) pour percer un tissu mou :
ladite tige allongée (10) possédant une extrémité proximale (12) supportant des membres (26, 27) qui peuvent être manipulés pour la manipulation dudit dispositif de serrage afin de serrer le tissu mou entre les mâchoires de serrage (20, 21) et pour manipuler ledit dispositif de perçage (30) afin de percer le tissu mou (40) lorsque ce dernier est serré entre lesdites mâchoires de serrage (20, 21), et pour tirer un fil de suture (15, 16) à travers le tissu mou percé (40) ;
**caractérisé en ce que** ladite tige allongée (10) est rigide entre son extrémité proximale (12) et son extrémité distale (11) ; et
**en ce que** ledit instrument chirurgical englobe en outre, à l'extrémité distale (11) de ladite tige allongée (10), une douille (13) qui est dimensionnée pour recevoir une ancre (14) qui doit venir s'implanter dans un alésage (43a, 43b, 43c) pratiqué dans l'os (43), un fil de suture (15, 16) que l'on doit faire passer à travers le tissu mou percé (40) et qui doit être rattaché à l'os (43) étant également fixé à ladite ancre (14).

2. Instrument chirurgical selon la revendication 1, dans lequel ledit dispositif de perçage (30) englobe une tête à crochet (32) possédant une extrémité distale pointue (33) pour percer le tissu (40) serré entre les mâchoires (20, 21) dudit dispositif de serrage au cours du mouvement de la tête à crochet (32) en direction distale.

3. Instrument chirurgical selon la revendication 2, dans lequel ladite tête à crochet (32) possède un profil en forme de crochet (34) espacé de son extrémité distale pointue (33) pour recevoir le fil de suture (15, 16) et pour tirer ce dernier à travers le tissu percé (40) au cours du mouvement de la tête à crochet (32) dans la direction proximale.

4. Instrument chirurgical selon la revendication 1, dans lequel ladite extrémité distale (12) de la tige allongée (10) comporte une fente (15e) s'étendant sur la trajectoire du mouvement du dispositif de perçage (30) lorsque celui-ci se déplace dans la direction proximale, pour la réception du fil de suture (15) qui doit être tiré à travers un trou formé dans le tissu (40) après le perçage via ledit dispositif de perçage (30).

5. Instrument chirurgical selon la revendication 4, dans lequel ladite extrémité distale (12) de la tige allongée (10) comporte une deuxième fente (16a) pour la réception d'un deuxième fil de suture (16) fixé à l'ancre (14), qui doit être tiré à travers un deuxième trou formé par ledit dispositif de perçage.

6. Instrument chirurgical selon la revendication 1, dans lequel lesdites mâchoires de serrage (20, 21) englobent une mâchoire fixe (20) fixée à ladite tige allongée (10) et une mâchoire pivotante (21) qui pivote en direction et à l'écart de ladite mâchoire fixe (20) pour prendre des positions de fermeture et d'ouverture par rapport à cette dernière.

7. Instrument chirurgical selon la revendication 6, dans lequel ledit dispositif de perçage (30) se déplace à travers un canal (19) formé dans ladite mâchoire fixe (20).

8. Instrument chirurgical selon la revendication 1, dans lequel l'axe longitudinal de ladite douille (14) à ladite extrémité distale (11) de la tige allongée (10) est parallèle à l'axe longitudinal de ladite tige allongée (10) ; et dans lequel l'extrémité proximale (12) de la tige allongée (10) englobe un doigt de commande (17) possédant un axe également parallèle à l'axe longitudinal de ladite tige allongée pour forcer par pression une ancre (14) dans ladite douille (13) à l'intérieur d'un alésage (43a) formé dans l'os (41).

9. Instrument chirurgical selon la revendication 1, dans lequel lesdits membres manipulables supportés à l'extrémité proximale (12) de ladite tige allongée (10) englobent : une première poignée (26) fixée à l'extrémité proximale (12) de ladite tige allongée (10) et une deuxième poignée (25) qui pivote à l'extrémité proximale (12) de ladite tige allongée et qui est couplée à une mâchoire (21) parmi lesdites mâchoires de serrage (20, 21) pour déplacer ladite mâchoire afin de prendre une position de fermeture et d'ouverture par rapport à ladite mâchoire fixe (20) lors du pivotement de ladite deuxième poignée (25) par rapport à ladite première poignée (26).

10. Instrument chirurgical selon la revendication 9, dans lequel on lesdits membres manipulables supportés à l'extrémité proximale de ladite tige allongée (10) englobent en outre un bras de levier (31) apte à pivoter en direction de ladite première poignée (26) et couplé audit dispositif de perçage (30) pour déplacer ce dernier dans les directions distale et proximale lors du pivotement dudit bras de levier (31) par rapport à ladite première poignée (26).

11. Instrument chirurgical selon la revendication 9, dans lequel lesdits membres manipulables supportés par ladite extrémité proximale de la tige allongée (10) englobent en outre un dispositif d'encliquetage (27) pour retenir de manière amovible ladite première poignée (26) et ladite deuxième poignée (25) dans une position de pivotement sélectionnée, l'une par rapport à l'autre.

12. Instrument chirurgical selon la revendication 1, dans lequel ladite tige allongée (10), lesdits dispositifs de serrage (20-23) et ledit dispositif de perçage (30) sont dimensionnés pour réparer une déchirure du labrum dans l'articulation d'une épaule.
